# EUROPEAN PATENT SPECIFICATION

(11) **EP 3 760 123 B1**
(45) Date of publication and mention of the grant of the patent: **17.12.2025**
(21) Application number: 19184711.0
(22) Date of filing: 05.07.2019
(51) Int. Cl.: A61B 5/16, A61B 5/0205, A61B 5/00, A61B 5/021, A61B 5/024, A61B 5/318, A61B 5/369, A61B 5/389, A61B 5/398, A61B 5/0531, A61B 5/11, A61B 5/145

(54) **A SYSTEM FOR ESTIMATING A STRESS CONDITION OF AN INDIVIDUAL**
SYSTEM ZUR SCHÄTZUNG EINES BELASTUNGSZUSTANDS EINES INDIVIDUUMS
SYSTÈME D'ESTIMATION D'UN ÉTAT DE STRESS D'UN INDIVIDU

(43) Date of publication of application: 06.01.2021
(73) Proprietor: IMEC vzw, 3001 Leuven (BE); Katholieke Universiteit Leuven KU Leuven Research & Development, 3000 Leuven (BE); Stichting IMEC Nederland, 6708 WH Wageningen (NL)
(72) Inventor: SMETS, Elena, 3001 Leuven (BE); RIOS VELAZQUEZ, Emmanuel, 3001 Leuven (BE); SCHIAVONE, Giuseppina, 3001 Leuven (BE)
(74) Representative: AWA Sweden AB

(56) References cited:
- US-A1- 2012 289 788
- US-A1- 2015 182 129
- US-A1- 2016 357 924

## Description

### Technical field

The present invention relates to a system and method for estimating a condition of a person. In particular, the present invention relates to estimating a stress condition of a human.

### Background

There is growing worldwide awareness of the problems caused by long-term stress, such as depression, burn-out and cardiovascular disorders. The number of workdays lost due to anxiety, stress and neurotic disorders is four times higher than the number of lost days due to other non-fatal injuries and illnesses. In the European Union, more than 40 million individuals are affected by work-related stress. Stress is one of the most commonly reported causes of occupational illness by workers and costs approximately 20 billion euros per year in lost productivity and medical expenses, as reported in Institute of Work, Health & Organisations, "Towards the development of a European framework for psychosocial risk management at the workplace", 2008. Stress comes in many flavors and has many aspects. Biological stress describes the physiological reaction to stressors or threats. Psychological stress is related to psychological, social reactions and consequences caused by stress. Stress can lead to diseases that influence the ability to work and affects the social environment such as the families of stressed persons. Biologically, stress is a strategy to address threatening situations and events to increase survival chances. For example, if an animal is confronted with an attack of a predator, stress triggers decision processes outside the usual channels, allows quicker reactions and, thus, increases the chances of survival.

Stress leads to various physiological responses in humans including increased adrenaline and cortisol levels, increased heart rate, dry mouth, dilated pupils, bladder relaxation, tunnel vision, shaking, flushed face, slowed digestion, hearing loss, and change in the electrical properties of the skin.

Stress and its physiological manifestation that can be measured by sensors is an utmost personal phenomenon. Each individual reacts differently to stress, and thus, the readings from sensors are different. For example, for one individual, stress might manifest itself in a permanently increased heart rate, whereas another individual might show variation in electrodermal response. Such a wide variety of possible physiological responses makes it difficult to develop a generalized stress estimator that can predict the stress level of all persons. Instead, it is required to tailor the estimator to each individual.

Moreover, each individual interprets his or her own stress level or stress condition differently. Currently, the common practice to detect stress condition such as mental health diseases is by using questionnaires. However, these are subjective, time-consuming and based on spot-checks only.

Further, none of the above physiological responses are exclusively caused by stress. Instead, there are various reasons that alter physiological signals in a similar way as stress does and other events or situations not relating to stress can yield physiological responses similar to stress. For example, the heart rate increases also if a person performs physical demanding activities, and the electrodermal properties of the skin are influenced by temperature and humidity of the surrounding air. To better estimate a physiological stress of an individual, both the perceived stress-level of the individual and the measured physiological parameters from the sensors should be taken into account.

US 2016/357924 A1 is a prior art example of a system for managing a risk of medication dependence that determines a conservative estimate of the time when the patient will become dependent on a medication as a function of a medication log.

US 2012/0289788 (Fujitsu Limited) discloses a method to annotate abnormal physiological moments, by asking the user to indicate a type of mood and intensity, so that a link can be stablished between abnormal physiological parameters and moods/activities and their intensity. In this document, an improved estimate of a physiological stress of an individual requires more measured physiological parameters using further sensors.

However, combining multiple sensors to a multimodal sensor stream is prone to errors and requires advanced and costly devices for collecting all required data. Moreover, since physiological responses and its connection to stress are individual, as described above, collecting more data does not necessarily result in a better estimation of stress. It would be desired to have a robust stress estimator, that is able to address those issues without cost-intensive data collection and labeling.

### Summary

The invention is defined by the subject matter of the independent claims. Particular embodiments of the invention are set out in the dependent claims.

It is an object of the invention to provide a system for estimating a physiological stress condition of an individual, which is robust and reliable, using low-complexity system without any computationally expensive calculations. It is further an object to provide a system which in an efficient way distributes functionality for performing the physiological stress estimation between units of the system.

According to an aspect of the present disclosure, there is provided a system for estimating a stress condition of an individual, the system comprising a mobile device and a network unit, the mobile device being connected to the network unit and to one or more sensors.

The mobile device comprises circuity configured to: for each occasion of a plurality of occasions:
- measure a set of physiological parameters using the one or more sensors, and transmit first data relating to the set of physiological parameters to the network unit; and
- prompt the individual to input a perceived stress-level for the occasion via a user interface of the mobile device and transmit second data relating to the perceived stress-level to the network unit.

As used herein, a mobile device refers to for example a smart phone, a smart watch, a virtual assistant or any other mobile device which an individual can interact with, using a user interface such as a graphical user interface (GUI) or voice commands etc. The mobile device may include one or more sensors or be connected to such sensor(s). In some embodiment, the mobile device is a sensor which also can receive user input, such as a more advanced pulse measuring unit or camera. The mobile device also comprises functionality for transmitting data to a network unit, e.g. comprising a wireless or wired transmitter.

As used herein, physiological parameters refer to parameters describing functions of the body of the individual, such as heart rate, blood pressure, body temperature, electrical properties of the skin, serum levels of various stress hormones, posture etc., Such parameters can be measured or determined by various types of sensors, as will be described further below.

The network unit comprises circuity configured to, for each occasion of the plurality of occasions:
- receive the first data from the mobile device, extracting a set of physiological parameters from the first data, and determine for the occasion a measured stress metric by applying a first predetermined stress metric function to at least the extracted set of physiological parameters;
- receive the second data the mobile device, extracting a perceived stress metric from the second data, and determine for the occasion a perceived stress-level by applying a second predetermined stress-metric function to at least the extracted perceived stress-level; and
- calculate a stress-level discrepancy based on a difference between the measured stress-metric and the perceived stress-metric;

The network unit is further configured to generate, based on the stress-level discrepancies calculated at the plurality of occasions and a stress-level discrepancy threshold, a feedback signal indicative of a stress condition of the individual.

As used herein, a network unit refers to a server, or similar network connected unit, which may be cloud based or a local physical unit. The network unit includes functionality for receiving data from the mobile device, e.g. a wired or wireless receiver. One or more processors (circuity) of the network unit are used for determining a measured stress metric and a perceived stress metric from the received data. The first and second predetermined stress metric functions are used to ensure that the two stress metrics are defined in a same scale, e.g. the Likert scale. In some embodiments, the second data received from the mobile device includes perceived stress-level which already are in the correct scale. In this case, the second predetermined stress metric function only defines an injective function which does not change the value of the perceived stress-level when determining the perceived metric. Consequently, the user interface of the mobile device may in some embodiments prompt the individual to input a perceived stress-level using a single value, e.g. five-point Likert scale (no stress to extreme stress).

As used herein, a stress condition may also be referred to as a chronic stress symptom, or a physiological chronic stress.

The first predetermined stress metric function depends on what types of physiological parameters that are measured by the sensors connected to the mobile device. Many known stress metric functions exist, and it is left to the implementor of the present invention to choose a suitable stress metric function.

When the two metrics are determined in the same scale, comparison between the two may advantageously be done.

In the prior art, improving the accuracy and reliability of a diagnoses of a stress condition have been accomplished (or at least tried to be accomplished) by improving the sensing of physiological parameters using more or better sensors, or by implementing more/better individual subjective input. This inevitably result in a more complex and possibly expensive system.

In the present disclosure, a low complexity system is presented, which provides a more accurate and reliable indication of a stress condition. Just collecting/measuring physiological parameters and using these for estimating stress does not provide a robust estimation of a physiological stress condition, since different individuals react differently to stress. Instead of collecting more data on each occasion or try to find the "perfect" physiological parameter for estimation a stress condition, discrepancies between the physiological measurements (measured stress-metric) and the perceived stress level (perceived stress-metric), when compared over time, can be advantageously used, according to the present description, to predict if an individual may experience a stress condition. This understanding can be used to provide a more accurate and reliable indication of a stress condition and be achieved with a comparably low-complexity system without any computationally expensive calculations. By using a mobile device for collecting the data needed, and letting a network device or unit determine, based on the collected data, if the individual is in risk of a stress condition using two predetermined stress-metric functions and a stress-level discrepancy threshold, a low complexity mobile device may be advantageously employed. Moreover, latency between providing the data to the network unit, and the network unit generating the feedback signal indicative of a stress condition of the individual may be reduced. It should be noted that the network unit may determine if the individual is in risk of a stress condition after each received first and second data (i.e. for each occasion), using e.g. a sliding window approach.

According to one embodiment, the network unit is configured to: upon generation of the feedback signal, transmit the feedback signal to the mobile device, wherein the mobile device is further configured to provide feedback to the individual based on the received feedback signal. For example, the feedback may comprise displaying or playing an alarm to the individual that the individual may risk developing a stress condition such as physiological chronic stress. In some embodiment, also if the network unit determines that the individual is not in risk of developing a stress condition, the individual is informed via the feedback provided by to the mobile device.

As described above, since latency in the system may be reduced, the individual may advantageously be informed, and possibly convey that information to a caretaker (a psychiatrist, medical doctor, coach, etc.,), without any significant delay from inputting the perceived stress-level to the mobile device.

According to some embodiments, the network unit is configured to, upon generation of the feedback signal, transmit the feedback signal to a device separate from the mobile device. In this embodiment, the feedback signal from the network unit may be routed to a device under control of another person with interest of the stress condition of the individual, e.g. a caretaker (a psychiatrist, medical doctor, coach, etc.,), an employer, a spouse, etc.,. This person may thus advantageously receive indication of the stress condition of the individual in near real time.

According to some embodiments, the circuity of the network unit is configured to generate the feedback signal indicating a stress condition in response to a threshold number of the calculated stress-level discrepancies exceeding the stress-level discrepancy threshold. In this embodiment, the network unit generates the feedback signal indicating a stress condition only after the stress-level discrepancy threshold has been exceeded a number of times. Consequently, the risk of an erroneous detection, for example due to a wrong input by the individual, or due to faulty sensor data, is reduced. It should be noted that the network unit may also in this embodiment determine if the individual is in risk of a stress condition after each received first and second data (i.e. for each occasion), using e.g. a sliding window approach.

According to some embodiments, the circuity of the network unit is configured to generate the feedback signal indicating a stress condition in response to an average of the calculated stress-level discrepancies exceeding the stress-level discrepancy threshold. In this embodiment, the network unit may compare an average of a number of stress-level discrepancies and then compare the average to the discrepancy threshold to decide whether to provide the feedback signal indicating a stress condition or not.

Consequently, the risk of an erroneous diagnosis, for example due to a wrong input by the individual, or due to faulty sensor data, may be reduced. It should be noted that the network unit may also in this embodiment determine if the individual is in risk of a stress condition after each received first and second data (i.e. for each occasion), using e.g. a sliding window approach.

According to some embodiments, the mobile device is configured to, for an occasion of a plurality of occasions, transmit the first data and the second data in separate transmissions, wherein each the first and second data further indicates a point in time of the occasion. Consequently, the first data may be transmitted as soon as the mobile device has received the corresponding set of physiological parameters from the one or more sensors, or when the bandwidth of the connection with the network unit allows such transmission. The second data may then be transmitted to the network device when the individual input the perceived stress-level, or when the bandwidth of the connection with the network unit allows such transmission. The point in time of the first and second data associated with a same occasion may be used to associate these with each other at the network unit.

The flexibility of the system may thus be increased. Moreover, the system is less sensitive to the mobile device not being in connection with the network unit all the time. Moreover, the network unit may take action if first data is received, but not second data, e.g. by alarming to a caretaker that the individual may be unable or not willing to input data regarding the perceived stress-level, which in itself may indicate a stress condition or other types of conditions. Alternatively or additionally, the network unit may take action if first data is not received within a significant period of time (1 hour, 10 hours, 24 hours, etc.,) e.g. by alarming to a care taker.

According to some embodiments, the mobile device is configured to, for an occasion of a plurality of occasions, transmit the first data and the second data in a same transmission. This embodiment may advantageously reduce the complexity at the network unit, since the network unit may assume that first and second data received in a same transmission from the mobile device correspond to a same occasion.

According to some embodiments, the mobile device is configured to encrypt the perceived stress-level and the set of physiological parameters, wherein the first data comprises the encrypted set of physiological parameters, and wherein the second data comprises the encrypted perceived stress-level. Advantageously, privacy of the data transmissions from the mobile device is increased.

According to some embodiments, one or more sensors are included in the mobile device, wherein the mobile device is configured to be worn in contact with the skin of the individual. Existing devices on the market such as smart watches or similar comprising sensors may thus be employed in the present embodiment. Physiological parameters measured by the sensors of the mobile device is thus included in the first data transmitted to the network unit.

According to some embodiments, one or more sensors are included in a second mobile device configured to be worn in contact the skin of the individual, wherein the mobile device is configured to be wirelessly connected to the second mobile device and to receive physiological parameters measured by the at least one sensor included in the second mobile device and include the received physiological parameters in the set of physiological parameters. The flexibility of the system may thus be increased, since also peripheral sensors may be employed.

According to some embodiments, one or more sensors are non-contact sensors wirelessly connected to the mobile device or included in the mobile device, wherein the mobile device is configured to include the physiological parameters measured by the one or more non-contact sensors in the set of physiological parameters. Examples of such sensors may include a camera, an accelerometer, a radar, a capacitive sensor, or an audio recognition device.

According to some embodiments, the one or more sensors comprises at least one from the list of: a galvanic skin response sensor, an electroencephalogram sensor, a photoplethysmogram sensor, a bio-impedance sensor, an electromyogram sensor, an electrooculogram sensor, an electrocardiogram sensor, a temperature sensor, an accelerometer, a camera, an audio recognition device, and a gyroscope. Other suitable sensors may be employed.

According to some embodiments, the circuity of the mobile device is further configured to, for each occasion of the plurality of occasions, transmit third data to the network unit, the third data comprising at least one from the list of: metadata relating to the individual, and metadata relating to the occasion of the plurality of occasions,
wherein the circuity of the network unit is further configured to, for each occasion of the plurality of occasions, receive the third data from the mobile device, extract the metadata from the third data, and use the metadata as input in at least one of the first and second predetermined stress-metric function to determine at least one of the measured stress metric and the perceived stress-metric.

By also including metadata such as age, place of living, social status, gender, sport habit, smoking habit, marital status, education, work situation, etc., of the individual, or environmental factors relating to the occasion where the first and second data is collected by the mobile device such as time of day, weather, sound level, location, social recreational activity, activity (working out, working, sitting still), commuting condition etc., detection of situational stress may be improved, and outliers in the sensors' data may be detected.

According to some embodiments, the system comprises a plurality of further mobile devices, wherein each of the further mobile devices being connected to a second network unit and to one or more sensors configured for measuring a set of physiological parameters of a respective further individual.

Each of the further individual belongs to a first group of individuals or a second group of individuals, wherein the individuals of the first group are classified as mentally healthy and the individuals of the second group are classified as mentally un-healthy based on a stress-related criterion.

In this embodiment, the circuity of the second network unit is further configured to calculate the stress-level discrepancy threshold in a model phase comprising:
for each individual of the first and second group of individuals:
   receive on a plurality of occasions first data relating a set of physiological parameters measured by the one or more sensors configured for measuring a set of physiological parameters of the individual, and for each occasion, extracting a set of physiological parameters from the first data, and determine a measured stress metric by applying the first predetermined stress metric function to at least the extracted set of physiological parameters;
   receive for each of the plurality of occasions second data relating to a user perceived stress-level of the individual, extracting a user perceived stress-level from the second data, and determine a perceived stress metric by applying the second predetermined stress metric function to at least the extracted user perceived stress-level;
   calculate for each of the plurality of occasions a stress level discrepancy representing a difference between the measured stress metric and the perceived stress metric, and associating the stress level discrepancy to group of the individual;
wherein the circuity is further configured to:
   calculate the stress-level discrepancy threshold based on the calculated stress level discrepancies for the first and the second group of individuals.

The second network unit is either configured to communicate the stress-level discrepancy threshold to the network unit (i.e. the second network unit is remote from the network unit), or the second network unit is comprised in the network unit

Advantageously, the network unit may use the functionality described in the above embodiments also for calculating the stress-level discrepancy threshold. In this modelling phase, physiological parameters and perceived stress-levels for individuals in two reference groups (one "healthy" and one "un-healthy") are collected. Subsequently, based on the results for each reference group, the stress-level discrepancy threshold is calculated.

According to some embodiments, the circuity of the network unit is configured to calculate the stress-level discrepancy threshold using one from the list of: a clustering algorithm, a mean square error metric, Euclidean distance, and statistical interquartile differences.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 schematically shows a mobile device according to embodiments,
Fig. 2 schematically shows by way of example the mobile device of figure 1 connected to a network unit,
Fig. 3 schematically shows by way of example a system of a plurality of mobile devices of figure 1 connected to the network unit of figure 2.
Fig. 4 shows a method for estimating physiological stress of an individual according to embodiments,
Fig. 5 shows a method for determining a stress-level discrepancy threshold according to embodiments.

### Detailed description

The present disclosure relates to a new methodology towards mental disease prevention and interception, i.e. detecting a disease before there are any symptoms. Although literature agrees that disease interception is key towards early interventions and reduction of healthcare costs, techniques are lacking. Currently, the common practice to detect mental health diseases is by using questionnaires. However, these are subjective, time-consuming and based on spot-checks only. Additionally, questionnaires are often only used when a patient goes to a therapist for treatment. This stage is already past the interception stage as the patient in this case noticed his/her complaints. Physiological signals could provide a continuous, objective monitoring techniques that can be applied for large population screenings. However, as described above, it is not clear how these physiological signals could differentiate between healthy and un-healthy subjects. In the present disclosure, an invention focusing on the discrepancy between self-reported health indicators and predicted health based on physiological sensing is disclosed as a tool for disease interception.

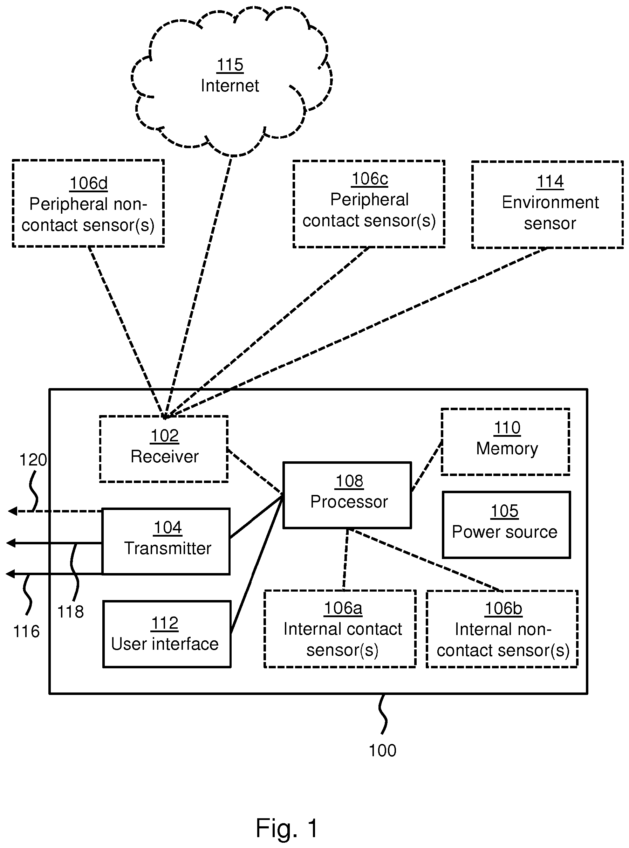

Figure 1 shows a mobile device 100 according to embodiments. The mobile device 100 is associated with an individual and configured to be used to aid the estimation of physiological stress of the individual.

The mobile device 100 may be any device having circuity configured to collect data from various internal 106a-b and/or externa (peripheral) sensors 106c-d configured to measure physiological parameters of the individual, as well as to other sources for information such as an environmental sensor 114 or the internet 115. The mobile device may for example be a smart watch, smart phone, a camera, or any internet of things (IoT) enabled device. The mobile device comprises a power source 105, for example a battery or a unit adapted to receive power via induction.

The circuity of the mobile device is configured to, for each occasion of a plurality of occasions, measure a set of physiological parameters of the individual using the one or more sensors 106a-d.

As described above, the sensors 106a-d may be internal or external to the mobile device 100. The sensors may be contact sensors 106a,c adapted to be worn in contact the skin of the individual and measuring physiological parameters such as heart rate, skin conductance, etc.,. Examples of such sensors include a galvanic skin response sensor, an electroencephalogram sensor, a photoplethysmogram sensor, a bio-impedance sensor, an electromyogram sensor, and an electrooculogram sensor, an electrocardiogram sensor, a temperature sensor.

The sensors may be non-contact sensors 106b,d such as a camera, an audio recognition device, or a gyroscope, or a touch screen, which are not adapted to be worn in contact the skin of the individual. Such sensors 106b,d may be configured to measure physiological parameters pertaining to for example movement patterns of an individual, posture of the individual, mood of the individual based on language analysis, etc.,.

A peripheral contact sensor 106c may be included in a second mobile device (such as a pulse measuring device, a smart watch, etc.,) configured to be worn in contact the skin of the individual. The mobile device 100 may be configured to be wirelessly connected to the second mobile device and to receive physiological parameters measured by the at least one sensor 106c included in the second mobile device using a receiver 102 of the mobile device 100.

A peripheral contact sensor 106c may also include a sensor implanted in the individual.

The mobile device may further be configured to be wirelessly connected to one or more non-contact sensors 106c and receive physiological parameters measured by the one or more non-contact sensors 106c using the receiver 102.

The mobile device may also comprise one or more sensor 106a, b, which may be contact sensor(s) 106a or non-contact sensor(s) 106b. The internal sensors 106a-b are also configured measure to a set of physiological parameters of the individual.

The device 100 comprises a processing unit, e.g. a processor 108, which determine first data 116 pertaining to the complete set of physiological parameters measured/collected by the one or more sensors 106a-d for a specific occasion. The first data 116 is transmitted by a transmitter 104 of the first device 100.

The transmitter 104 and the receiver 102 may be separate units or form a single unit, i.e. a transceiver.

The mobile device 100 is further configured to, for each occasions of the plurality of occasions, prompt the individual to input a perceived stress-level for the occasion via a user interface 112 of the mobile device 100. The mobile device 100 may prompt the individual using for example vibrations of the mobile device, sounds of the mobile device, light emitted by the mobile device or using any other suitable way of prompting.

The user interface 112 may for example be a graphical user interface where the user can input the perceived stress-level, or a voice recognition user interface such that the individual can input the perceived stress-level by voice. Any other suitable user interface may be employed.

The processing unit, e.g. the processor 108, of the mobile device determines second data 118 relating to the perceived stress-level for the specific occasion. The second data 118 is transmitted by a transmitter 104 of the first device 100.

The mobile device 100 may further be configured to, for each occasion of the plurality of occasions, collect or retrieve metadata relating to the individual, and/or metadata relating to the occasion of the plurality of occasions. The processor may form third data 120 from the metadata and the transmitter 104 may then transmit the third data.

The metadata may be measured by an environmental sensor 114 connected to the mobile device, for example a light sensor 114, a weather sensor 114, and/or a microphone 114, which can collect metadata relating to the occasion.

The metadata may also be retrieved from the internet 115. Such metadata may comprise data relating to the stock market, sports results, weather, the political climate, etc.,.

The metadata may also be retrieved from a memory 110 of the mobile device and comprise metadata relating to the individual such as age, social status, weight, demographical data, etc.,.

The mobile device 100 may comprise an internal clock (not shown in the figures) to keep track of time and coordinate the collection/retrieval of physiological parameters, the perceived stress-level and optionally the metadata.

The mobile device may receive data at regular intervals from peripheral sensors 106c-d by own motion of the peripheral sensors 106c-d,or request such data from the sensors 106c-d at regular intervals.

The first 116, second 118, and optionally the third 120 data relating to a specific occasion may be transmitted in a single transmission, or in different transmissions. The first, second and third data may further indicate a point in time of the occasion and/or identification data pertaining to the individual. The mobile device, e.g. the processor 108, may be configured to encrypt any of the transmitted data 116, 118, 120. In other words, the mobile device 100 may be configured to encrypt the perceived stress-level and the set of physiological parameters (and optionally the metadata), wherein the first data 116 comprises the encrypted set of physiological parameters, and/or the second data 118 comprises the encrypted perceived stress-level, and/or the third data 120 comprises the encrypted metadata. Any encryption method may be used. For example, an asymmetric encryption method may be used, where the mobile device 100 comprises the public key for encryption, which facilitates easy updating of software of a plurality of mobile devices.

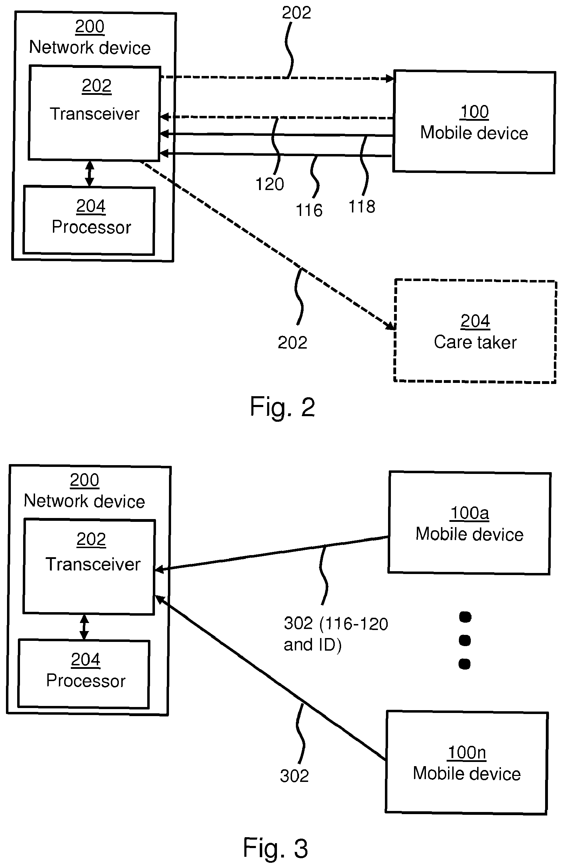

Figure 2 shows a system including the mobile device 100 of figure 1 wirelessly connected to a network unit 200. The network unit 200 is configured to receive the first 116, second 118 and optionally the third 120 data from the mobile device 100 using e.g. a transceiver 202 (or a separate receiver).

The network unit comprises circuity (e.g. one or more processors 204) for processing the received data 116, 118, 120. In other words, the network unit 200 comprising circuity configured to, for each occasion of the plurality of occasions: receive the first data 116 from the mobile device 100 and extracting (optionally decrypting) a set of physiological parameters from the first data. Similarly, the network unit 200 comprising circuity configured to, for each occasion of the plurality of occasions, receive the second data 118 the mobile device, and extracting (optionally decrypting) a perceived stress level from the second data 118.

Optionally, the network unit 200 comprising circuity configured to, for each occasion of the plurality of occasions, receive the third data 120 the mobile device, and extracting (optionally decrypting) metadata from the third data 120.

For each occasion, a measured stress metric is determined, by applying a first predetermined stress metric function to at least the extracted set of physiological parameters. The first predetermined metric function may be implemented using a logistic regression, a SVM, a decision tree, a random forest, a neural network, hierarchical Bayesian, hidden Markov models, etc.,.

The processor 204 is thus configured to determine measured stress-metric for the individual and for the occasion *t*: S _{(m_ind, t)} = F1(p1, p2...), where F1 is the predetermined stress metric function, p1, p2... are the physiological parameters extracted from the first data 116. In some embodiments, also metadata (m1, m2...) from the third data 120 is included, i.e. S _{(m_ind, t)} = F1(p1, p2..., m1, m2...).

For each occasion, a perceived stress metric is determined, by applying a second predetermined stress metric function to at least the perceived stress level.

The processor 204 is thus configured to determine perceived stress-metric for the individual and for the occasion *t*: S _{(p_ind,t)} = F2(PSL), where F2 is the predetermined stress metric function, and PSL is the perceived stress-level from the second data 118. In some embodiments, also metadata (m1, m2...) from the third data 120 is included, i.e. S _{(p_ind,t)} = F2(PSL, m1, m2...).

As described above, F2 may according to some embodiments only be used to make sure that the the two stress metrics S _{(m_ind, t)}, S_{(p_ind,t)} are defined in a same scale, e.g. the Likert scale.

For each occasion *t*, a stress-level discrepancy is calculated Δ_{(ind, t)} = S_{(m_ind,t)} - S_{(p_ind,t).} Based on the calculated discrepancies Δ_{ind} at the plurality of occasions (*t1...tn*) and a stress-level discrepancy threshold *DT,* the processor generates a feedback signal *FS* indicative of a stress condition of the individual.

FS = F3(Δ_{(ind, t1)}... Δ_{(ind, tn)}, DT), where F3 is a function for determining if the individual may be in risk of developing a stress condition or not.

According to some embodiments of the invention, the circuity of the network unit is configured to generate the feedback signal indicating a stress condition in response to a threshold number of the calculated stress-level discrepancies exceeding the stress-level discrepancy threshold. The threshold number may in some embodiments depend on the variance of the plurality of discrepancies, or be a static threshold such as 50%, 66%, etc., of the number of discrepancies.

In other embodiments of the invention, the circuity of the network unit is configured to generate the feedback signal indicating a stress condition in response to an average of the calculated stress-level discrepancies exceeding the stress-level discrepancy.

The network unit 200 may, upon generation of the feedback signal, transmit (sing the transceiver 202 or a separate transmitter) the feedback signal 202 to the mobile device 202. The mobile device may in this case be configured to provide feedback to the individual based on the received feedback signal. Alternatively, or additionally, the network unit is configured to, upon generation of the feedback signal, transmit the feedback signal to a device 204 separate from the mobile device. In the example of figure 2, the device 204 is associated with a caretaker of the individual, but other stakeholders such as a spouse, a parent or an employer may also receive the feedback signal.

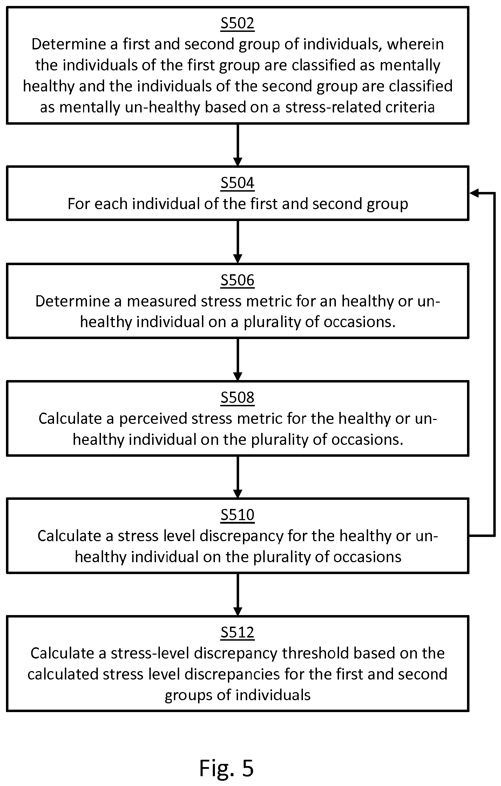

Figure 3 shows an embodiment where the network unit 200 further is configured to determine the stress-level discrepancy threshold *DT.* Such functionality will be described in conjunction with figure 5. In this embodiment, the network unit is connected to a plurality of further mobile devices 100a...n. Each of the further mobile devices 100a...n is connected to one or more sensors configured for measuring a set of physiological parameters of a respective further individual. Each of the further individuals belong to a first group of individuals or a second group of individuals. The individuals of the first group are classified S502 as mentally healthy and the individuals of the second group are classified as mentally un-healthy based on a stress-related criterion. For example, a self-test questionnaire (such as a perceived stress scale (PSS) test, or a depression, anxiety, stress scale, DASS, test) may be used to classify the individuals as healthy or at risk.

The network unit 200 is in this embodiment configured to, for each individual of the first and second group of individuals S504:
- receive on a plurality of occasions first data 302 relating a set of physiological parameters measured by the one or more sensors configured for measuring a set of physiological parameters of the individual, and for each occasion, extracting the set of physiological parameters from the first data, and determine S506 a measured stress metric by applying the first predetermined stress metric function to at least the extracted set of physiological parameters;
- receive for each of the plurality of occasions second data 302 relating to a user perceived stress-level of the individual, extracting a user perceived stress-level from the second data, and determine S508 a perceived stress metric by applying the second predetermined stress metric function to at least the extracted user perceived stress-level;
- calculate S508 for each of the plurality of occasions a stress level discrepancy representing a difference between the measured stress metric and the perceived stress metric and associating the stress level discrepancy to group of the individual.

In figure 3, for ease of explanation, the data 302 received from each of the mobile devices 100a...n is intended to comprise the first, second and optionally the third data as described in conjunction with figures 1-2. To keep track of, at the network unit, which individual that is associated with which received data 302, the data 302 advantageously comprises identification data as well. The information of which group a specific individual belongs to may be included in the data 302 or may be determined by the network unit using e.g. identification data in the received data 302 and a table of mappings between individuals and groups stored in the network unit 200.

Using the stress-level discrepancies calculated for the first and second group of individuals, for the plurality of occasions, the stress-level discrepancy threshold is calculated S512.

For example, a joint discrepancy may be calculated for the healthy and the un-healthy group of individuals, whereby the stress-level discrepancy threshold can be calculated based on the two joint discrepancies. The circuity of the network unit 200 may configured to calculate the stress-level discrepancy threshold using one from the list of: a clustering algorithm, a mean square error metric, a F₁ score metric, an accuracy metric, and Cohen's kappa matric. In other words, the assumption is that the discrepancy (i.e. false positives and/or false negatives) will be smaller for healthy subjects and larger the more severe the condition of the patient (i.e. the physiological response does not represent the perceived health correctly). E.g. the clustering can be used to identify a threshold stress-level discrepancy threshold to alarm patients at risk and/or caregivers. In some embodiments, a gradual indicator may be employed and included in the feedback signal (202 in figure 2) which may represent the distance to the centroid of the clusters, or the distance to the threshold stress-level discrepancy.

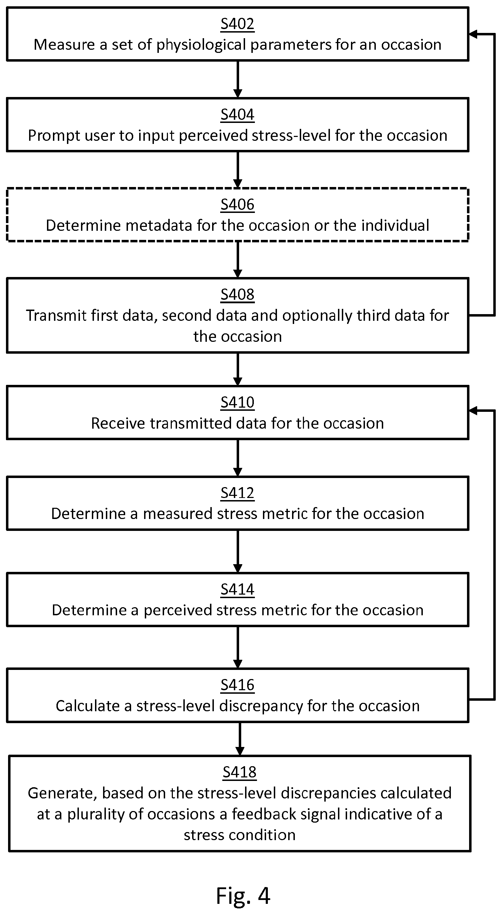

It should be noted that the embodiment of figure 3 is just by way of example. In figure 3 it is exemplified that it is the same network device 200 that generates a feedback signal indicative of a stress condition (e.g. according to figure 4) of an individual and calculates the stress-level discrepancy threshold (e.g. according to figure 5). However, in other embodiments, the network unit of figure 2 is instead configured to receive the stress-level discrepancy threshold from a remote (second) network unit configure to calculate the stress-level discrepancy threshold. In other words, the network unit of figure 2 and the network unit of figure 3 may be located in the same network (or may be located in another network). In the later case, the network unit of figure 2 and the (second) network unit of figure 3 exchange the stress-level discrepancy threshold when needed.

Figure 4 shows according to embodiments a method for estimating physiological stress of an individual in a system comprising a mobile device and a network unit, the mobile device being connected to the network unit and to one or more sensors. The method comprises the steps of:
for each occasion of a plurality of occasions:
measuring S402, by the mobile device, a set of physiological parameters using the one or more sensors, and transmitting S408 first data relating to the set of physiological parameters to the network unit;
prompting S404 the individual to input a perceived stress-level for the occasion via a user interface of the mobile device, and transmitting S408 second data relating to the perceived stress-level to the network unit,

The method may optionally comprise for each occasion of a plurality of occasions: determining S406, by the mobile device, metadata relating to the individual, and/or to the occasion of the plurality of occasions and transmitting S408 third data comprising the metadata to the network unit.

The steps S402, S404, optionally S406 and S408 are repeated for each occasion of the plurality of occasions.

The method further comprises, for each occasion of the plurality of occasions, receiving S410 the first data from the mobile device, extracting a set of physiological parameters from the first data, and determining S412 for the occasion a measured stress metric by applying a first predetermined stress metric function to at least the extracted set of physiological parameters. Optionally, the metadata is also employed as described above for determining S412 the measured stress metric.

The method further comprises, for each occasion of the plurality of occasions, receiving S410 the second data from the mobile device, extracting a perceived stress level from the second data, and determining S414 for the occasion a perceived stress-metric by applying a second predetermined stress-metric function to at least the extracted perceived stress-level. Optionally, the metadata is also employed as described above for determining S414 the perceived stress metric.

The method further comprises, for each occasion of the plurality of occasions, calculating S416 a stress-level discrepancy based on a difference between the measured stress-metric and the perceived stress-metric.

The steps S410, S412, S414, S416 are repeated for each occasion of the plurality of occasions.

The method further comprises the step of generating S418, based on the calculated stress-level discrepancies calculated at the plurality of occasions and a stress-level discrepancy threshold, a feedback signal indicative of a stress condition of the individual.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system for estimating a stress condition of an individual, the system comprising a mobile device (100) and a network unit (200), the mobile device (100) being connected to the network unit (200) and to one or more sensors (106),
the mobile device (100) comprising circuity configured to:
for each occasion of a plurality of occasions:
measure a set of physiological parameters using the one or more sensors (106), and transmit first data (116) relating to the set of physiological parameters to the network unit (200); and
prompt the individual to input a perceived stress-level for the occasion via a user interface (112) of the mobile device (100), and transmit second data (118) relating to the perceived stress-level to the network unit (200);
wherein the network unit (200) comprises circuity configured to,
for each occasion of the plurality of occasions:
receive the first data (116) from the mobile device (100), extracting a set of physiological parameters from the first data (116), and determine for the occasion a measured stress metric by applying a first predetermined stress metric function to at least the extracted set of physiological parameters;
receive the second data (118) the mobile device (100), extracting a perceived stress level from the second data (118), and determine for the occasion a perceived stress-metric by applying a second predetermined stress-metric function to at least the extracted perceived stress-level; and
calculate a stress-level discrepancy based on a difference between the measured stress-metric and the perceived stress-metric;
and;
the network unit (200) is further configured to generate a feedback signal (202) indicative of a stress condition of the individual, **characterised in that** the feedback signal (202) represents the output of a function, wherein the input to the function comprises the stress-level discrepancies calculated at the plurality of occasions and a stress-level discrepancy threshold, such that the feedback signal (202) indicates a stress condition in response to a threshold number of the calculated stress-level discrepancies exceeding the stress-level discrepancy threshold or an average of the calculated stress-level discrepancies exceeding the stress-level discrepancy threshold.

2. The system according to claim 1, wherein the network unit (200) is configured to:
Upon generation of the feedback signal (202), transmit the feedback signal (202) to the mobile device (100); and
wherein the mobile device (100) is further configured to provide feedback to the individual based on the received feedback signal (202).

3. The system according to any one of claims 1-2, wherein the network unit (200) is configured to:
upon generation of the feedback signal (202), transmit the feedback signal (202) to a device separate from the mobile device (100).

4. The system according to any one of claims 1-3, wherein the mobile device (100) is configured to, for an occasion of a plurality of occasions, transmit the first data (116) and the second data (118) in separate transmissions, wherein each the first and second data (116;118) further indicates a point in time of the occasion.

5. The system according to any one of claims 1-3, wherein the mobile device (100) is configured to, for an occasion of a plurality of occasions, transmit the first data (116) and the second data (118) in a same transmission.

6. The system according to any one of claims 1-5, wherein the mobile device (100) is configured to encrypt the perceived stress-level and the set of physiological parameters, wherein the first data (116) comprises the encrypted set of physiological parameters, and wherein the second data (118) comprises the encrypted perceived stress-level.

7. The system according to any one of claims 1-6, wherein one or more sensors (106) are included in the mobile device (100), and wherein the mobile device (100) is configured to be worn in contact with the skin of the individual.

8. The system according to any one of claims 1-7, wherein one or more sensors (106) are included in a second mobile device configured to be worn in contact the skin of the individual, and wherein the mobile device (100) is configured to be wirelessly connected to the second mobile device and to receive physiological parameters measured by the at least one sensor (106) included in the second mobile device and include the received physiological parameters in the set of physiological parameters.

9. The system according to any one of claims 1-8, wherein one or more sensors (106) are non-contact sensors wirelessly connected to the mobile device (100) or included in the mobile device (100), and wherein the mobile device (100) is configured to include the physiological parameters measured by the one or more non-contact sensors in the set of physiological parameters.

10. The system according to any one of claims 1-9,
wherein the circuity of the mobile device (100) is further configured to, for each occasion of the plurality of occasions, transmit third data (120) to the network unit (200), the third data (120) comprising at least one from the list of: metadata relating to the individual, and metadata relating to the occasion of the plurality of occasions,
wherein the circuity of the network unit (200) is further configured to, for each occasion of the plurality of occasions, receive the third data (120) from the mobile device (100), extract the metadata from the third data (120), and use the metadata as input in at least one of the first and second predetermined stress-metric function to determine at least one of the measured stress metric and the perceived stress-metric.

11. The system according to any one of claims 1-10, wherein the one or more sensors (106) comprises at least one from the list of: a galvanic skin response sensor, an electroencephalogram sensor, a photoplethysmogram sensor, a bio-impedance sensor, an electromyogram sensor, an electrooculogram sensor, an electrocardiogram sensor, an accelerometer, a camera, an audio recognition device, and a gyroscope.

12. The system according to any one of claims 1-11, wherein the system comprises a plurality of further mobile devices (100a-100n), wherein each of the further mobile devices is connected to a second network unit and to one or more sensors configured for measuring a set of physiological parameters of a respective further individual, wherein each of the further individuals belongs to a first group of individuals or a second group of individuals, wherein the individuals of the first group are classified as mentally healthy and the individuals of the second group are classified as mentally un-healthy based on a stress-related criteria, wherein the circuity of the second network unit is further configured to calculate the stress-level discrepancy threshold in a model phase comprising:
for each individual of the first and second group of individuals:
receive, on a plurality of occasions, first data (116) relating a set of physiological parameters measured by the one or more sensors (106) configured for measuring a set of physiological parameters of the individual, and for each occasion, extracting the set of physiological parameters from the first data (116), and determine a measured stress metric by applying the first predetermined stress metric function to at least the extracted set of physiological parameters;
receive, for each of the plurality of occasions, second data (118) relating to a user perceived stress-level of the individual, extracting a user perceived stress-level from the second data (118), and determine a perceived stress metric by applying the second predetermined stress metric function to at least the extracted user perceived stress-level;
calculate, for each of the plurality of occasions, a stress level discrepancy representing a difference between the measured stress metric and the perceived stress metric, and associating the stress level discrepancy to group of the individual;
wherein the circuity is further configured to:
calculate the stress-level discrepancy threshold based on the calculated stress level discrepancies for the first and the second group of individuals,
wherein the second network unit is configured to communicate the stress-level discrepancy threshold to the network unit, or wherein the network unit comprises the second network unit.

13. The system of claim 12, wherein the circuity of the network unit is configured to calculate the stress-level discrepancy threshold using at least one from the list of: a clustering algorithm, a mean square error metric, Euclidean distance, and statistical interquartile difference.

## Patentansprüche

1. System zum Schätzen eines Stresszustands einer Person, wobei das System eine Mobilvorrichtung (100) und eine Netzwerkeinheit (200) umfasst, wobei die Mobilvorrichtung (100) mit der Netzwerkeinheit (200) und mit einem oder mehreren Sensoren (106) verbunden ist,
wobei die Mobilvorrichtung (100) eine Schaltung umfasst, die eingerichtet ist zum:
für jeden Anlass von mehreren Anlässen:
Messen eines Satzes physiologischer Parameter unter Verwendung des einen oder der mehreren Sensoren (106) und Senden erster Daten (116), die sich auf den Satz physiologischer Parameter beziehen, an die Netzwerkeinheit (200); und
Auffordern der Person, einen wahrgenommenen Stresspegel für den Anlass über eine Benutzerschnittstelle (112) der Mobilvorrichtung (100) einzugeben, und Senden zweiter Daten (118) in Bezug auf den wahrgenommenen Stresspegel an die Netzwerkeinheit (200);
wobei die Netzwerkeinheit (200) eine Schaltung umfasst, die eingerichtet ist zum:
für jeden Anlass von mehreren Anlässen:
Empfangen der ersten Daten (116) von der Mobilvorrichtung (100), Extrahieren eines Satzes physiologischer Parameter aus den ersten Daten (116), und Bestimmen, für den Anlass, einer gemessenen Stresskennzahl durch Anwenden einer ersten zuvor festgelegten Stresskennzahlfunktion auf mindestens den extrahierten Satz physiologischer Parameter;
Empfangen der zweiten Daten (118) von der Mobilvorrichtung (100), Extrahieren eines wahrgenommenen Stresspegels aus den zweiten Daten (118), und Bestimmen, für den Anlass, einer wahrgenommenen Stresskennzahl durch Anwenden einer zweiten zuvor festgelegten Stresskennzahlfunktion auf mindestens den extrahierten wahrgenommenen Stresspegel; und
Berechnen einer Stresspegeldiskrepanz auf der Grundlage einer Differenz zwischen der gemessenen Stresskennzahl und der wahrgenommenen Stresskennzahl;
und
wobei die Netzwerkeinheit (200) des Weiteren dazu eingerichtet ist, ein Rückmeldungssignal (202) zu generieren, das einen Stresszustand der Person angibt,
**dadurch gekennzeichnet, dass** das Rückmeldungssignal (202) die Ausgabe einer Funktion darstellt, wobei die Eingabe in die Funktion die bei mehreren Anlässen berechneten Stresspegeldiskrepanzen und eine Stresspegeldiskrepanzschwelle umfasst, dergestalt, dass das Rückmeldungssignal (202) einen Stresszustand in Reaktion darauf angibt, dass eine Schwellenzahl der berechneten Stresspegeldiskrepanzen die Stresspegeldiskrepanzschwelle überschreitet oder ein Durchschnitt der berechneten Stresspegeldiskrepanzen die Stresspegeldiskrepanzschwelle überschreitet.

2. System nach Anspruch 1, wobei die Netzwerkeinheit (200) eingerichtet ist zum:
nach Generierung des Rückmeldungssignals (202), Senden des Rückmeldungssignals (202) an die Mobilvorrichtung (100); und
wobei die Mobilvorrichtung (100) des Weiteren dazu eingerichtet ist, der Person auf der Grundlage des empfangenen Rückmeldungssignals (202) eine Rückmeldung zu geben.

3. System nach einem der Ansprüche 1 und 2, wobei die Netzwerkeinheit (200) eingerichtet ist zum:
nach Generierung des Rückmeldungssignals (202), Senden des Rückmeldungssignals (202) an eine von der Mobilvorrichtung (100) getrennte Vorrichtung.

4. System nach einem der Ansprüche 1-3, wobei die Mobilvorrichtung (100) dazu eingerichtet ist, für einen Anlass von mehreren Anlässen die ersten Daten (116) und die zweiten Daten (118) in separaten Übertragungen zu senden, wobei sowohl die ersten als auch die zweiten Daten (116; 118) des Weiteren einen Zeitpunkt des Anlasses angeben.

5. System nach einem der Ansprüche 1-3, wobei die Mobilvorrichtung (100) dazu eingerichtet ist, für einen Anlass von mehreren Anlässen die ersten Daten (116) und die zweiten Daten (118) in einer selben Übertragung zu senden.

6. System nach einem der Ansprüche 1-5, wobei die Mobilvorrichtung (100) dazu eingerichtet ist, den wahrgenommenen Stresspegel und den Satz physiologischer Parameter zu verschlüsseln, wobei die ersten Daten (116) den verschlüsselten Satz physiologischer Parameter umfassen und wobei die zweiten Daten (118) den verschlüsselten wahrgenommenen Stresspegel umfassen.

7. System nach einem der Ansprüche 1-6, wobei ein oder mehrere Sensoren (106) in der Mobilvorrichtung (100) enthalten sind, und wobei die Mobilvorrichtung (100) dazu eingerichtet ist, in Kontakt mit der Haut der Person getragen zu werden.

8. System nach einem der Ansprüche 1-7, wobei ein oder mehrere Sensoren (106) in einer zweiten Mobilvorrichtung enthalten sind, die dazu eingerichtet ist, in Kontakt mit der Haut der Person getragen zu werden, und wobei die Mobilvorrichtung (100) dazu eingerichtet ist, drahtlos mit der zweiten Mobilvorrichtung verbunden zu werden und physiologische Parameter zu empfangen, die durch den mindestens einen Sensor (106) gemessen werden, der in der zweiten Mobilvorrichtung enthalten ist, und die empfangenen physiologischen Parameter in den Satz physiologischer Parameter einzubinden.

9. System nach einem der Ansprüche 1-8, wobei ein oder mehrere Sensoren (106) kontaktlose Sensoren sind, die drahtlos mit der Mobilvorrichtung (100) verbunden oder in der Mobilvorrichtung (100) enthalten sind, und wobei die Mobilvorrichtung (100) dazu eingerichtet ist, die durch den einen oder die mehreren kontaktlosen Sensoren gemessenen physiologischen Parameter in den Satz physiologischer Parameter einzubinden.

10. System nach einem der Ansprüche 1-9,
wobei die Schaltung der Mobilvorrichtung (100) des Weiteren dazu eingerichtet ist, für jeden Anlass der mehreren Anlässe dritte Daten (120) an die Netzwerkeinheit (200) zu senden, wobei die dritten Daten (120) mindestens eines aus der Liste umfassen, die Metadaten, die sich auf die Person beziehen, und Metadaten, die sich auf den Anlass der mehreren Anlässe beziehen, umfasst,
wobei die Schaltung der Netzwerkeinheit (200) des Weiteren dazu eingerichtet ist, für jeden Anlass der mehreren Anlässe die dritten Daten (120) von der Mobilvorrichtung (100) zu empfangen, die Metadaten aus den dritten Daten (120) zu extrahieren, und die Metadaten als Eingabe in mindestens einer der ersten und der zweiten zuvor festgelegten Stresskennzahlfunktion zu verwenden, um mindestens eine der gemessenen Stresskennzahl und der wahrgenommenen Stresskennzahl zu bestimmen.

11. System nach einem der Ansprüche 1-10, wobei der eine oder die mehreren Sensoren (106) mindestens eines aus der Liste umfassen, die einen Hautleitwertsensor, einen Elektroenzephalogrammsensor, einen Photoplethysmogrammsensor, einen Bioimpedanzsensor, einen Elektromyogrammsensor, einen Elektrookulogrammsensor, einen Elektrokardiogrammsensor, einen Beschleunigungsmesser, eine Kamera, eine Audioerkennungsvorrichtung und ein Gyroskop umfasst.

12. System nach einem der Ansprüche 1-11, wobei das System mehrere weitere Mobilvorrichtungen (100a-100n) umfasst, wobei jede der weiteren Mobilvorrichtungen mit einer zweiten Netzwerkeinheit und mit einem oder mehreren Sensoren, die zum Messen eines Satzes physiologischer Parameter einer jeweiligen weiteren Person eingerichtet sind, verbunden ist, wobei jede der weiteren Personen zu einer ersten Gruppe von Personen oder einer zweiten Gruppe von Personen gehört, wobei, basierend auf einem stressbezogenen Kriterium, die Personen der ersten Gruppe als psychisch gesund klassifiziert sind und die Personen der zweiten Gruppe als psychisch krank klassifiziert sind, wobei die Schaltung der zweiten Netzwerkeinheit des Weiteren dazu eingerichtet ist, die Stresspegeldiskrepanzschwelle in der Modellphase zu berechnen, umfassend:
für jede Person der ersten und der zweiten Gruppe von Personen:
Empfangen, bei mehreren Anlässen, erster Daten (116), die sich auf einen Satz physiologischer Parameter beziehen, die durch den einen oder die mehreren Sensoren (106), die zum Messen eines Satzes physiologischer Parameter der Person eingerichtet sind, gemessen wurden, und für jeden Anlass, Extrahieren des Satzes physiologischer Parameter aus den ersten Daten (116), und Bestimmen einer gemessenen Stresskennzahl durch Anwenden der ersten zuvor festgelegten Stresskennzahlfunktion auf mindestens den extrahierten Satz physiologischer Parameter;
Empfangen, für jeden der mehreren Anlässe, zweiter Daten (118), die sich auf einen durch einen Benutzer wahrgenommenen Stresspegel der Person beziehen, Extrahieren eines durch einen Benutzer wahrgenommenen Stresspegels aus den zweiten Daten (118), und Bestimmen einer wahrgenommenen Stresskennzahl durch Anwenden der zweiten zuvor festgelegten Stresskennzahlfunktion auf mindestens den extrahierten durch den Benutzer wahrgenommenen Stresspegel;
Berechnen, für jeden der mehreren Anlässe, einer Stresspegeldiskrepanz, die eine Differenz zwischen der gemessenen Stresskennzahl und der wahrgenommenen Stresskennzahl darstellt, und Verknüpfen der Stresspegeldiskrepanz mit der Gruppe der Person;
wobei die Schaltung des Weiteren eingerichtet ist zum:
Berechnen der Stresspegeldiskrepanzschwelle auf der Grundlage der berechneten Stresspegeldiskrepanzen für die erste und die zweite Gruppe von Personen,
wobei die zweite Netzwerkeinheit dazu eingerichtet ist, die Stresspegeldiskrepanzschwelle an die Netzwerkeinheit zu übermitteln, oder wobei die Netzwerkeinheit die zweite Netzwerkeinheit umfasst.

13. System nach Anspruch 12, wobei die Schaltung der Netzwerkeinheit dazu eingerichtet ist, die Stresspegeldiskrepanzschwelle unter Verwendung mindestens eines aus einer Liste zu berechnen, die einen Clusterbildungsalgorithmus, eine Kennzahl eines mittleren quadratischen Fehlers, einen euklidischen Abstand und eine statistische Interquartilsdifferenz umfasst.

## Revendications

1. Système d'estimation du niveau de stress d'un individu, le système comprenant un dispositif mobile (100) et une unité de réseau (200), le dispositif mobile (100) étant connecté à l'unité de réseau (200) et à un ou plusieurs capteurs (106),
le dispositif mobile (100) comprenant des circuits configurés pour :
pour chaque occasion d'une pluralité d'occasions :
mesurer un ensemble de paramètres physiologiques à l'aide du ou des capteurs (106) et transmettre des premières données (116) relatives à l'ensemble de paramètres physiologiques à l'unité de réseau (200) ; et
inviter l'individu à saisir un niveau de stress perçu pour l'occasion via une interface d'utilisateur (112) du dispositif mobile (100) et transmettre des deuxièmes données (118) relatives au niveau de stress perçu à l'unité de réseau (200) ;
dans lequel l'unité de réseau (200) comprend des circuits configurés pour à chaque occasion de la pluralité d'occasions ;
recevoir les premières données (116) du dispositif mobile (100), extraire un ensemble de paramètres physiologiques des premières données (116) et déterminer, pour l'occasion, un niveau de stress mesuré en appliquant une première fonction de niveau de stress prédéterminée à au moins l'ensemble de paramètres physiologiques extraits ;
recevoir les deuxièmes données (118) du dispositif mobile (100), extraire un niveau de stress perçu des deuxièmes données (118) et déterminer, pour l'occasion, un niveau de stress perçu en appliquant une deuxième fonction de niveau de stress prédéterminée au niveau de stress perçu extrait ; et
calculer un écart de niveau de stress sur la base d'une différence entre le niveau de stress mesuré et le niveau de stress perçu ; et
l'unité de réseau (200) est en outre configurée pour générer un signal de rétroaction (202) indiquant un état de stress de l'individu,
**caractérisé en ce que** le signal de rétroaction (202) représente la sortie d'une fonction, dans lequel la saisie de la fonction comprend les écarts de niveau de stress calculés à plusieurs occasions et un seuil d'écart de niveau de stress, de sorte que le signal de rétroaction (202) indique un état de stress en réponse à un nombre seuil d'écarts de niveau de stress calculés dépassant le seuil d'écart de niveau de stress ou à une moyenne des écarts de niveau de stress calculés dépassant le seuil d'écart de niveau de stress.

2. Système selon la revendication 1, dans lequel l'unité de réseau (200) est configurée pour :
lors de la génération du signal de rétroaction (202), transmettre le signal de rétroaction (202) au dispositif mobile (100) ; et
dans lequel le dispositif mobile (100) est en outre configuré pour fournir une rétroaction à l'utilisateur en fonction du signal de rétroaction reçu (202).

3. Système selon une quelconque des revendications 1 et 2, dans lequel l'unité de réseau (200) est configurée pour :
lors de la génération du signal de rétroaction (202), transmettre le signal de rétroaction (202) à un dispositif distinct du dispositif mobile (100).

4. Système selon une quelconque des revendications 1 à 3, dans lequel le dispositif mobile (100) est configuré pour, à une occasion d'une pluralité d'occasions, transmettre les premières données (116) et les deuxièmes données (118) lors de transmissions distinctes, dans lequel chacune des premières et deuxièmes données (116 ; 118) indique un instant précis de l'occasion.

5. Système selon une quelconque des revendications 1 à 3, dans lequel le dispositif mobile (100) est configuré pour, à une occasion d'une pluralité d'occasions, transmettre les premières données (116) et les deuxièmes données (118) lors d'une même transmission.

6. Système selon une quelconque des revendications 1 à 5, dans lequel le dispositif mobile (100) est configuré pour chiffrer le niveau de stress perçu et l'ensemble de paramètres physiologiques, dans lequel les premières données (116) comprennent l'ensemble chiffré de paramètres physiologiques et dans lequel les deuxièmes données (118) comprennent le niveau de stress perçu chiffré.

7. Système selon une quelconque des revendications 1 à 6, dans lequel un ou plusieurs capteurs (106) sont intégrés au dispositif mobile (100), et dans lequel le dispositif mobile (100) est conçu pour être porté au contact de la peau de l'individu.

8. Système selon une quelconque des revendications 1 à 7, dans lequel un ou plusieurs capteurs (106) sont intégrés à un deuxième dispositif mobile conçu pour être porté au contact de la peau de l'individu et dans lequel le dispositif mobile (100) est configuré pour être connecté sans fil au deuxième dispositif mobile et pour recevoir des paramètres physiologiques mesurés par au moins un capteur (106) intégré au deuxième dispositif mobile et inclure les paramètres physiologiques reçus dans l'ensemble de paramètres physiologiques.

9. Système selon une quelconque des revendications 1 à 8, dans lequel un ou plusieurs capteurs (106) sont des capteurs sans contact connectés sans fil au dispositif mobile (100) ou intégrés à celui-ci, et dans lequel le dispositif mobile (100) est configuré pour inclure les paramètres physiologiques mesurés par le ou les capteurs sans contact dans l'ensemble de paramètres physiologiques.

10. Système selon une quelconque des revendications 1 à 9,
dans lequel le circuit du dispositif mobile (100) est en outre configuré pour, à chaque occasion parmi plusieurs occasions, transmettre des données tierces (120) à l'unité de réseau (200), les données tierces (120) comprenant au moins une donnée parmi la liste de : métadonnées relatives à l'individu et métadonnées relatives à l'occasion parmi plusieurs occasions,
dans lequel le circuit de l'unité de réseau (200) est configuré pour, à chaque occasion parmi plusieurs occasions, recevoir les données tierces (120) du dispositif mobile (100), extraire les métadonnées des données tierces (120) et utiliser les métadonnées comme entrée dans au moins une des première et deuxième fonctions de mesure du stress prédéterminées afin de déterminer au moins une de la mesure du stress mesuré et la mesure du stress perçu.

11. Système selon une quelconque des revendications 1 à 10, dans lequel le ou les capteurs (106) comprennent au moins un élément parmi la liste de : un capteur de réponse cutanée galvanique cutanée, un capteur d'électroencéphalogramme, un capteur de photopléthysmogramme, un capteur de bioimpédance, un capteur d'électromyogramme, un capteur d'électrooculogramme, un capteur d'électrocardiogramme, un accéléromètre, une caméra, un dispositif de reconnaissance audio et un gyroscope.

12. Système selon une quelconque des revendications 1 à 11, comprenant une pluralité de dispositifs mobiles supplémentaires (100a-100n), dans lequel chacun des dispositifs mobiles supplémentaires est connecté à une deuxième unité de réseau et à un ou plusieurs capteurs configurés pour mesurer un ensemble de paramètres physiologiques d'un individu supplémentaire respectif, dans lequel chacun des individus supplémentaires appartient à un premier groupe d'individus ou à un deuxième groupe d'individus, dans lequel les individus du premier groupe sont classés comme mentalement sains et les individus du deuxième groupe comme mentalement malades, sur la base de critères liés au stress, dans lequel le circuit de la deuxième unité de réseau est en outre configuré pour calculer le seuil d'écart du niveau de stress dans une phase de modélisation comprenant :
pour chaque individu des premier et deuxième groupes d'individus :
la réception, à plusieurs occasions, de premières données (116) relatives à un ensemble de paramètres physiologiques mesurés par le ou les capteurs (106) configurés pour mesurer un ensemble de paramètres physiologiques de l'individu et, pour chaque occasion, l'extraction de l'ensemble de paramètres physiologiques des premières données (116) et la détermination d'un niveau de stress mesuré en appliquant une première fonction de niveau de stress prédéterminée à au moins l'ensemble extrait de paramètres physiologiques ;
la réception, pour chacune des occasions, des deuxièmes données (118) relatives au niveau de stress perçu par l'utilisateur, l'extraction d'un niveau de stress perçu par l'utilisateur des deuxièmes données (118), et la détermination d'un niveau de stress perçu en appliquant la deuxième fonction de niveau de stress prédéterminée à au moins le niveau de stress perçu par l'utilisateur extrait ;
le calcul, pour chacune des occasions, d'un écart de niveau de stress représentant une différence entre le niveau de stress mesuré et le niveau de stress perçu, et l'association de cet écart de niveau de stress au groupe de l'individu ;
dans lequel le circuit est en outre configuré pour :
calculer le seuil d'écart de niveau de stress sur la base des écarts de niveau de stress calculés pour le premier et le deuxième groupe d'individus,
dans lequel la deuxième unité de réseau est configurée pour communiquer le seuil d'écart de niveau de stress à l'unité de réseau ou dans lequel l'unité de réseau comprend la deuxième unité de réseau.

13. Système selon la revendication 12, dans lequel le circuit de l'unité de réseau est configuré pour calculer le seuil d'écart de niveau de stress en utilisant au moins un parmi la liste de: un algorithme de clustering, une métrique d'erreur quadratique moyenne, la distance euclidienne et la différence interquartile statistique.
